# EUROPÄISCHE PATENTSCHRIFT

(11) **EP 4 265 189 B1**
(45) Veröffentlichungstag und Bekanntmachung des Hinweises auf die Patenterteilung: **10.09.2025**
(21) Anmeldenummer: 22199104.5
(22) Anmeldetag: 30.09.2022
(51) Int. Cl.: A61B 6/03, A61B 6/00

(54) **COMPUTERTOMOGRAPHIEGERÄT UND VERFAHREN ZUM AUSFÜHREN VON TRANSLATIONSBEWEGUNGEN VON GANTRYTEILEN EINES COMPUTERTOMOGRAPHIEGERÄTS**
COMPUTED TOMOGRAPHY DEVICE AND METHOD FOR CARRYING OUT TRANSLATION MOTIONS OF GANTRY PARTS OF A COMPUTED TOMOGRAPHY DEVICE
APPAREIL DE TOMOGRAPHIE ASSISTÉE PAR ORDINATEUR ET PROCÉDÉ POUR EFFECTUER DES MOUVEMENTS DE TRANSLATION DE PARTIES DE PORTIQUE D'UN APPAREIL DE TOMOGRAPHIE ASSISTÉE PAR ORDINATEUR

(43) Veröffentlichungstag der Anmeldung: 25.10.2023
(73) Patentinhaber: Siemens Healthineers AG, 91301 Forchheim (DE)
(72) Erfinder: Eichner, Christoph, 91052 Erlangen (DE); Jensch, Aurel, 91330 Eggolsheim (DE); Löwen, Viktor, 53757 Sankt Augustin (DE)
(74) Vertreter: Siemens Healthineers Patent Attorneys

(56) Entgegenhaltungen:
- EP-A1- 3 342 348
- CN-A- 114 098 790
- CN-A- 114 947 922

## Beschreibung

Die Erfindung betrifft ein Computertomographiegerät. Die Erfindung betrifft ferner ein Verfahren zum Ausführen von Translationsbewegungen von Gantryteilen eines Computertomographiegeräts.

Um ein Untersuchungsobjekt mit Hilfe eines Computertomographiegeräts zu untersuchen, kann beispielsweise eine Scanbewegung durchgeführt werden. Dabei werden das Untersuchungsobjekt und ein Projektionsdatenakquisitionssystem des Computertomographiegeräts relativ zueinander translatorisch bewegt, während mittels des Projektionsdatenakquisitionssystems Projektionsdaten von einem Untersuchungsbereich des Untersuchungsobjekts erfasst werden, insbesondere um einen mehrschichtigen medizinischen Bilddatensatz erzeugen zu können.

In bestimmten Situation kann es vorteilhaft sein, wenn die Scanbewegung durchgeführt werden kann, während das Untersuchungsobjekt relativ zu einer Umgebung des Computertomographiegeräts, insbesondere relativ zu einem Untersuchungsraum, ruht. Dafür wird das Projektionsdatenakquisitionssystem des Computertomographiegeräts relativ zu der Umgebung des Computertomographiegeräts translatorisch bewegt, während mittels des Projektionsdatenakquisitionssystems Projektionsdaten von dem Untersuchungsbereich des Untersuchungsobjekts erfasst werden.

Beispielsweise kann dafür ein Gantryteil des Computertomographiegeräts, der das Projektionsdatenakquisitionssystem aufweist, relativ zu einem Gantryteil des Computertomographiegeräts, der relativ zu dem Untersuchungsobjekt und relativ zu der Umgebung des Computertomographiegeräts ruht, translatorisch bewegt werden.

Als Stand der Technik sind hierbei die CN 114 098 790 A und CN 114 947 922 A zu nennen.

Die Erfindung hat die Aufgabe, für ein Computertomographiegerät eine Lagerung eines ersten Gantryteils relativ zu einem zweiten Gantryteil und eine Lagerung eines dritten Gantryteils relativ zu dem zweiten Gantryteil derart bereitzustellen, dass der dritte Gantryteil einer Translationsbewegung des ersten Gantryteils relativ zu dem zweiten Gantryteil folgt, wenn eine erste Verbindung, welche den dritten Gantryteil relativ zu dem ersten Gantryteil lösbar fixiert, hergestellt ist, und dass während der Translationsbewegung des ersten Gantryteils relativ zu dem zweiten Gantryteil der dritte Gantryteil relativ zu dem zweiten Gantryteil ruht, wenn die erste Verbindung gelöst ist und eine zweite Verbindung, welche den dritten Gantryteil relativ zu dem zweiten Gantryteil lösbar fixiert, hergestellt ist, und dabei eine Kraft zu verringern, welche während der Translationsbewegung des ersten Gantryteils relativ zu dem zweiten Gantryteil auf ein Lösen der zweiten Verbindung, welche den dritten Gantryteil relativ zu dem zweiten Gantryteil lösbar fixiert, hinwirkt. Jeder Gegenstand eines unabhängigen Anspruchs löst diese Aufgabe. In den abhängigen Ansprüchen sind weitere vorteilhafte Aspekte der Erfindung berücksichtigt.

Die Erfindung betrifft ein Computertomographiegerät, aufweisend eine Gantry mit einem ersten Gantryteil, einem zweiten Gantryteil und einem dritten Gantryteil, wobei der erste Gantryteil einen Rotor mit einem Projektionsdatenakquisitionssystem aufweist und mittels einer ersten Linearführung derart relativ zu dem zweiten Gantryteil bewegbar gelagert ist, dass eine Translationsbewegung des ersten Gantryteils relativ zu dem zweiten Gantryteil ausgeführt werden kann, insbesondere entlang der ersten Linearführung ausgeführt werden kann, wobei der erste Gantryteil eine erste Verbindungseinheit aufweist und der zweite Gantryteil eine zweite Verbindungseinheit aufweist.

Der dritte Gantryteil weist eine dritte Verbindungseinheit auf, die derart zu der ersten Verbindungseinheit korrespondierend ausgebildet ist, dass mittels der ersten Verbindungseinheit und der dritten Verbindungseinheit eine erste Verbindung hergestellt werden kann, welche den dritten Gantryteil relativ zu dem ersten Gantryteil lösbar fixiert, wobei der dritte Gantryteil der Translationsbewegung des ersten Gantryteils relativ zu dem zweiten Gantryteil folgt, wenn die erste Verbindung hergestellt ist.

Der dritte Gantryteil weist eine vierte Verbindungseinheit auf, die derart zu der zweiten Verbindungseinheit korrespondierend ausgebildet ist, dass mittels der zweiten Verbindungseinheit und der vierten Verbindungseinheit eine zweite Verbindung hergestellt werden kann, welche den dritten Gantryteil relativ zu dem zweiten Gantryteil lösbar fixiert, wobei während der Translationsbewegung des ersten Gantryteils relativ zu dem zweiten Gantryteil der dritte Gantryteil relativ zu dem zweiten Gantryteil ruht, wenn die erste Verbindung gelöst ist und die zweite Verbindung hergestellt ist.

Insbesondere kann vorgesehen sein, dass während der Translationsbewegung des ersten Gantryteils relativ zu dem zweiten Gantryteil der dritte Gantryteil relativ zu dem ersten Gantryteil ruht, wenn die erste Verbindung hergestellt ist.

Die erste Linearführung weist ein Schienensystem und ein Wagensystem, welches mit dem Schienensystem zusammenwirkt, auf. Die Erfindung sieht vor, dass der erste Gantryteil das Wagensystem aufweist und dass der zweite Gantryteil das Schienensystem aufweist. Die Gantry kann beispielsweise ferner einen Linearantrieb zum Antreiben der Translationsbewegung des ersten Gantryteils relativ zu dem zweiten Gantryteil aufweisen. Der Linearantrieb kann beispielsweise eine Gewindespindel und einen Gewindetrieb, der mit der Gewindespindel zusammenwirkt, aufweisen. Insbesondere kann vorgesehen sein, dass der erste Gantryteil den Gewindetrieb aufweist und/oder dass der zweite Gantryteil die Gewindespindel aufweist.

Eine Ausführungsform sieht vor, dass die erste Verbindung formschlüssig ist und/oder dass die zweite Verbindung kraftschlüssig ist. Durch eine kraftschlüssige zweite Verbindung kann die Gefahr einer Klemmung im Vergleich zu einer grundsätzlich auch möglichen formschlüssigen zweiten Verbindung verringert werden. Insbesondere kann vorgesehen sein, dass sich die zweite Verbindungseinheit an einer Oberfläche des zweiten Gantryteils befindet und/oder dass sich die vierte Verbindungseinheit an einer Oberfläche des dritten Gantryteils befindet.

Eine Ausführungsform sieht vor, dass die zweite Verbindung auf einer magnetischen Anziehung zwischen der zweiten Verbindungseinheit und der vierten Verbindungseinheit basiert. Die zweite Verbindung kann beispielsweise magnetisch sein. Durch die Verwendung einer kraftschlüssigen magnetischen zweiten Verbindung kann die Oberfläche der Gantry im Bereich der zweiten Verbindungseinheit und/oder im Bereich der vierten Verbindungseinheit reinigungsoptimiert gestaltet werden.

Eine Ausführungsform sieht vor, dass die zweite Verbindungseinheit einen Elektromagneten aufweist und/oder dass die vierte Verbindungseinheit ein ferromagnetisches Material aufweist. Die vierte Verbindungseinheit kann beispielsweise eine ferromagnetische Platte, beispielsweise aus Stahl, aufweisen. Beispielsweise kann ein Herstellen der zweiten Verbindung durch ein Einschalten des Elektromagneten erfolgen, wenn sich das ferromagnetische Material der vierten Verbindungseinheit in einem Wirkungsbereich des Elektromagneten befindet. Beispielsweise kann ein Lösen der zweiten Verbindung durch ein Ausschalten des Elektromagneten erfolgen.

Eine Ausführungsform sieht vor, dass die erste Verbindungseinheit einen Riegel und einen Aktor zum Verschieben des Riegels aufweist und/oder dass die dritte Verbindungseinheit zur formschlüssigen Aufnahme des Riegels eingerichtet ist. Die erste Verbindungseinheit kann beispielsweise ein Hubzylinder, insbesondere in Form eines Elektrozylinders, Pneumatikzylinders oder Hydraulikzylinders, sein.

Es sind somit keine aktiven Komponenten auf dem dritten Gantryteil erforderlich, um die erste Verbindung und die zweite Verbindung herstellen bzw. lösen zu können. Somit ist für diese Zwecke eine Übertragung von Steuerungsdaten und/oder elektrischer Energie zum dritten Gantryteil nicht erforderlich.

Die Erfindung sieht vor, dass der dritte Gantryteil mittels einer zweiten Linearführung relativ zu dem ersten Gantryteil bewegbar gelagert ist und dass die zweite Linearführung parallel zu der ersten Linearführung ausgerichtet ist.

Eine Ausführungsform sieht vor, dass die erste Verbindung auf einer Verriegelung basiert, welche den dritten Gantryteil formschlüssig gegen eine Translationsbewegung des dritten Gantryteils entlang der zweiten Linearführung relativ zu dem ersten Gantryteil sichert.

Die Erfindung sieht vor, dass die zweite Linearführung einen Satz von zueinander parallelen Führungswellen und einen Satz von Kugelbuchsen aufweist und dass jede Führungswelle des Satzes von zueinander parallelen Führungswellen in einer entsprechenden Kugelbuchse des Satzes von Kugelbuchsen für eine Längsbewegung dieser Führungswelle gelagert ist.

Insbesondere kann vorgesehen sein, dass der erste Gantryteil den Satz von Kugelbuchsen aufweist und dass der dritte Gantryteil den Satz von zueinander parallelen Führungswellen aufweist.

Alternativ dazu kann vorgesehen sein, dass der dritte Gantryteil den Satz von Kugelbuchsen aufweist und dass der erste Gantryteil den Satz von zueinander parallelen Führungswellen aufweist. Grundsätzlich ist es auch möglich, dass der erste Gantryteil eine erste Führungswelle des Satzes von zueinander parallelen Führungswellen und eine erste Kugelbuchse des Satzes von Kugelbuchsen aufweist und dass der dritte Gantryteil eine zu der ersten Kugelbuchse korrespondierende zweite Führungswelle des Satzes von zueinander parallelen Führungswellen und eine zu der ersten Führungswelle korrespondierende zweite Kugelbuchse des Satzes von Kugelbuchsen aufweist.

Dadurch kann dann, wenn während der Translationsbewegung des ersten Gantryteils relativ zu dem zweiten Gantryteil der dritte Gantryteil relativ zu dem zweiten Gantryteil ruht, die Reibung zwischen dem dritten Gantryteil und dem ersten Gantryteil verringert werden. Außerdem kann auch dann, wenn während der Translationsbewegung des ersten Gantryteils relativ zu dem zweiten Gantryteil der dritte Gantryteil relativ zu dem zweiten Gantryteil ruht, das Gewicht des dritten Gantryteils von dem zweiten Gantryteil im Wesentlichen über den Satz von zueinander parallelen Führungswellen und den Satz von Kugelbuchsen aufgenommen werden, sodass es nicht erforderlich ist, die zweite Verbindung für ein Halten eines wesentlichen Teils des Gewichts des dritten Gantryteils auszulegen. Insbesondere kann der Elektromagnet der zweiten Verbindungseinheit somit weniger leistungsstark ausgeführt werden.

Dadurch wird ein ruhiger und gleichmäßiger Lauf des dritten Gantryteils relativ zu dem ersten Gantryteil dann, wenn während der Translationsbewegung des ersten Gantryteils relativ zu dem zweiten Gantryteil der dritte Gantryteil relativ zu dem zweiten Gantryteil ruht, ermöglicht.

Eine Ausführungsform sieht vor, dass der dritte Gantryteil wenigstens eine Führungswelle des Satzes von zueinander parallelen Führungswellen aufweist, welche eine Einkerbung zur formschlüssigen Aufnahme eines entsprechend angeordneten Riegels der ersten Verbindungseinheit aufweist. Die wenigstens eine Führungswelle des Satzes von zueinander parallelen Führungswellen kann somit die dritte Verbindungseinheit bilden.

Damit ist es nicht erforderlich, zusätzlich zu den Führungswellen separate Verriegelungs-Gegenstücke einzubauen.

Die Erfindung sieht vor, dass die Gantry eine Öffnung aufweist, wobei die Öffnung derart ausgebildet ist, dass ein Untersuchungsobjekt entlang einer Systemachse der Gantry in die Öffnung eingeführt werden kann, und dass die Translationsbewegung des ersten Gantryteils relativ zu dem zweiten Gantryteil entlang der Systemachse erfolgt.

Insbesondere kann vorgesehen sein, dass die erste Linearführung parallel zu der Systemachse ausgerichtet ist und/oder dass die zweite Linearführung parallel zu der Systemachse ausgerichtet ist. Insbesondere kann vorgesehen sein, dass jede Führungswelle des Satzes von zueinander parallelen Führungswellen zu der Systemachse parallel ist und/oder dass für jede Führungswelle des Satzes von zueinander parallelen Führungswellen die Längsbewegung dieser Führungswelle entlang der Systemachse erfolgt.

Insbesondere kann vorgesehen sein, dass der erste Gantryteil im Wesentlichen ringförmig um die Systemachse herum angeordnet ist und/oder dass der dritte Gantryteil im Wesentlichen ringförmig um die Systemachse herum angeordnet ist.

Insbesondere kann vorgesehen sein, dass die zueinander parallelen Führungswellen des Satzes von zueinander parallelen Führungswellen um die Systemachse herum in einem im Wesentlichen gleichmäßigen auf die Systemachse bezogenen Winkelabstand voneinander angeordnet sind. Beispielsweise können drei zueinander parallele Führungswellen vorgesehen sein, die voneinander jeweils einen auf die Systemachse bezogenen Winkelabstand von 120 Grad haben.

Insbesondere kann vorgesehen sein, dass der erste Gantryteil eine Drehlagerung und eine Tragstruktur aufweist und dass der Rotor mittels der Drehlagerung mit der Tragstruktur verbunden und relativ zu der Tragstruktur um die Systemachse drehbar gelagert ist.

Das Untersuchungsobjekt kann beispielsweise ein Körperteil, insbesondere ein menschliches oder tierisches Körperteil, oder ein Phantom sein. Das Untersuchungsobjekt kann insbesondere ein Kopf eines Menschen sein. Das Computertomographiegerät kann insbesondere als Kopf-Computertomographiegerät und/oder als mobiles Computertomographiegerät ausgebildet sein.

Die Erfindung sieht vor, dass der erste Gantryteil eine Rückseite einer Verkleidung der Gantry aufweist, wobei die Rückseite der Verkleidung der Gantry eine Rückseite der Öffnung ringförmig umgibt, und dass der dritte Gantryteil eine Vorderseite einer Verkleidung der Gantry aufweist, wobei die Vorderseite der Verkleidung der Gantry eine Vorderseite der Öffnung ringförmig umgibt.

Eine Ausführungsform sieht vor, dass die vierte Verbindungseinheit an der Vorderseite der Verkleidung der Gantry befestigt ist. Insbesondere kann vorgesehen sein, dass sich die ferromagnetische Platte der vierten Verbindungseinheit im Wesentlichen parallel zu einer Oberfläche der Vorderseite der Verkleidung der Gantry flächig erstreckt und/oder dass eine Plattenebene der ferromagnetischen Platte zu der Systemachse im Wesentlichen senkrecht, insbesondere senkrecht, ist.

Die Erfindung betrifft ferner ein Verfahren zum Ausführen von Translationsbewegungen von Gantryteilen eines erfindungsgemä-ßen Computertomographiegeräts, das Verfahren umfassend:
- ein Herstellen der ersten Verbindung,
- ein erstes Ausführen der Translationsbewegung des ersten Gantryteils relativ zu dem zweiten Gantryteil in eine erste Richtung, wobei der dritte Gantryteil der Translationsbewegung des ersten Gantryteils relativ zu dem zweiten Gantryteil folgt,
- ein erstes Ausführen der Translationsbewegung des ersten Gantryteils relativ zu dem zweiten Gantryteil in eine zweite Richtung, wobei der dritte Gantryteil der Translationsbewegung des ersten Gantryteils relativ zu dem zweiten Gantryteil folgt,
- ein Herstellen der zweiten Verbindung,
- ein Lösen der ersten Verbindung,
- ein zweites Ausführen der Translationsbewegung des ersten Gantryteils relativ zu dem zweiten Gantryteil in die erste Richtung, wobei der dritte Gantryteil relativ zu dem zweiten Gantryteil ruht.

Insbesondere kann die zweite Richtung zu der ersten Richtung entgegengesetzt sein. Insbesondere kann vorgesehen sein, dass die erste Richtung zu der Systemachse parallel ist und/oder dass die zweite Richtung zu der Systemachse parallel ist.

Insbesondere kann vorgesehen sein, dass nach dem ersten Ausführen der Translationsbewegung des ersten Gantryteils relativ zu dem zweiten Gantryteil in die erste Richtung und vor dem ersten Ausführen der Translationsbewegung des ersten Gantryteils relativ zu dem zweiten Gantryteil in die zweite Richtung ein Untersuchungsobjekt derart relativ zu der Gantry positioniert wird, dass durch die Translationsbewegung des ersten Gantryteils relativ zu dem zweiten Gantryteil in die zweite Richtung das Untersuchungsobjekt entlang der Systemachse der Gantry in die Öffnung eingeführt wird, wobei während der Translationsbewegung des ersten Gantryteils relativ zu dem zweiten Gantryteil in die zweite Richtung das Untersuchungsobjekt relativ zu dem zweiten Gantryteil ruht.

Insbesondere kann vorgesehen sein, dass während des zweiten Ausführens der Translationsbewegung des ersten Gantryteils relativ zu dem zweiten Gantryteil in die erste Richtung Projektionsdaten von einem Untersuchungsbereich des Untersuchungsobjekts mittels des Projektionsdatenakquisitionssystems erfasst werden.

Eine Ausführungsform sieht vor, dass die erste Verbindungseinheit einen Riegel und einen Aktor zum Verschieben des Riegels aufweist, wobei die dritte Verbindungseinheit zur formschlüssigen Aufnahme des Riegels eingerichtet ist, wobei die erste Verbindung hergestellt wird, indem der Riegel mittels des Aktors hin zu der dritten Verbindungseinheit verschoben und mittels der dritten Verbindungseinheit formschlüssig aufgenommen wird, wobei die erste Verbindung gelöst wird, indem der Riegel mittels des Aktors weg von der dritten Verbindungseinheit verschoben wird.

Das Herstellen der ersten Verbindung kann beispielsweise durch eine Verriegelung erfolgen, welche den dritten Gantryteil formschlüssig gegen eine Translationsbewegung des dritten Gantryteils entlang der zweiten Linearführung relativ zu dem ersten Gantryteil sichert. Das Lösen der ersten Verbindung kann beispielsweise durch eine Entriegelung erfolgen, welche die Translationsbewegung des dritten Gantryteils entlang der zweiten Linearführung relativ zu dem ersten Gantryteil freigibt.

Eine Ausführungsform sieht vor, dass die zweite Verbindungseinheit einen Elektromagneten aufweist, wobei die vierte Verbindungseinheit ein ferromagnetisches Material aufweist, wobei durch das erste Ausführen der Translationsbewegung des ersten Gantryteils relativ zu dem zweiten Gantryteil in die zweite Richtung das ferromagnetische Material der vierten Verbindungseinheit in einen Wirkungsbereich des Elektromagneten gebracht wird, während der Elektromagnet ausgeschaltet ist, wobei die zweite Verbindung hergestellt wird, indem der Elektromagnet eingeschaltet wird.

Die zweite Verbindung kann beispielsweise gelöst werden, indem der Elektromagnet ausgeschaltet wird. Insbesondere kann vorgesehen sein, dass durch das erste Ausführen der Translationsbewegung des ersten Gantryteils relativ zu dem zweiten Gantryteil in die zweite Richtung die vierte Verbindungseinheit bis zu einem formschlüssigen Anschlag an die zweite Verbindungseinheit angenähert wird. Dadurch kann eine ruckartige Bewegung des ersten Gantryteils beim Einschalten des Elektromagneten sowie das damit verbundene Risiko für Beschädigungen und/oder Quetschungen vermieden werden.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Die Verwendung der unbestimmten Artikel "ein" bzw. "eine" schließt nicht aus, dass das betroffene Merkmal auch mehrfach vorhanden sein kann.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.
Die Fig. 1 zeigt ein Computertomographiegerät in Form eines mobilen Kopf-Computertomographiegeräts mit einer Kopfschale und einer Körperstützvorrichtung.
Die Fig. 2 zeigt ein Computertomographiegerät in einem ersten Betriebszustand.
Die Fig. 3 zeigt eine Schnittansicht des Computertomographiegeräts in dem ersten Betriebszustand.
Die Fig. 4 zeigt das Computertomographiegerät in einem zweiten Betriebszustand.
Die Fig. 5 zeigt eine Schnittansicht des Computertomographiegeräts in einem Zwischenzustand.
Die Fig. 6 zeigt eine Schnittansicht des Computertomographiegeräts in dem zweiten Betriebszustand.
Die Fig. 7 zeigt das Computertomographiegerät in einem dritten Betriebszustand.
Die Fig. 8 zeigt eine Schnittansicht des Computertomographiegeräts in dem dritten Betriebszustand.
Die Fig. 9 zeigt ein Ablaufdiagramm eines Verfahrens zum Ausführen von Translationsbewegungen von Gantryteilen eines Computertomographiegeräts

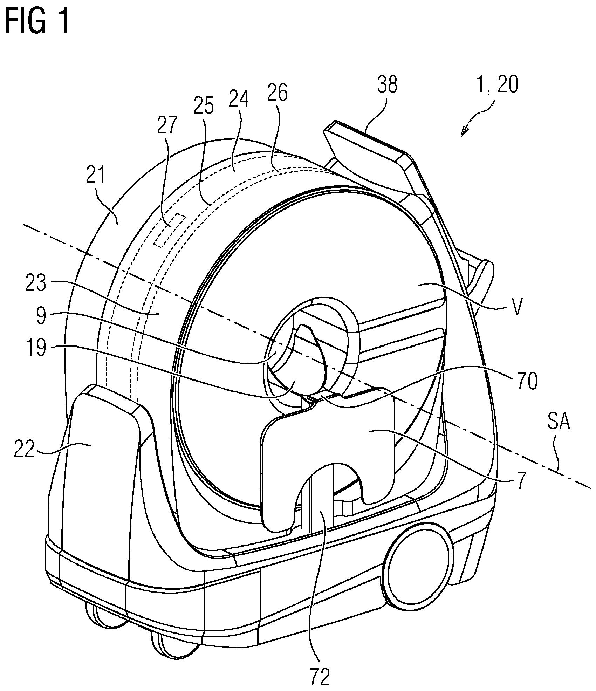

Die Fig. 1 zeigt ein Computertomographiegerät 1 in Form eines mobilen Kopf-Computertomographiegeräts mit einer Kopfschale 19 und einem Schulterbrett 7. Das Computertomographiegerät 1 weist die Gantry 20 mit einem ersten Gantryteil 21, einem zweiten Gantryteil 22 und einem dritten Gantryteil 23, wobei der erste Gantryteil 21 einen Rotor 24 mit einem Projektionsdatenakquisitionssystem 27 aufweist und mittels einer ersten Linearführung derart relativ zu dem zweiten Gantryteil 22 bewegbar gelagert ist, dass eine Translationsbewegung des ersten Gantryteils 21 relativ zu dem zweiten Gantryteil 22 ausgeführt werden kann, insbesondere entlang der ersten Linearführung ausgeführt werden kann.

Der erste Gantryteil 21 weist eine Drehlagerung 25 und eine Tragstruktur 26 auf, wobei der Rotor 24 mittels der Drehlagerung 25 mit der Tragstruktur 26 verbunden und relativ zu der Tragstruktur 26 um die Systemachse SA drehbar gelagert ist. Das Schulterbrett 7 ist mittels der Schwenkvorrichtung 70 mit der Haltevorrichtung 72 verbunden und relativ zu der Gantry 20 um eine zu der Systemachse SA senkrechte Schwenkachse schwenkbar gelagert. Die Systemachse SA ist horizontal und parallel zu der ersten Richtung Z1 und parallel zu der zweiten Richtung Z2. Der zweite Gantryteil 22 weist ein Fahrwerk für eine horizontale Transportbewegung der Gantry 20 auf. Die Gantry 20 weist ferner den berührungsempfindlichen Bildschirm 38 zur Bedienung des Computertomographiegeräts 1 auf.

Die Gantry 20 weist eine Öffnung 9 auf, wobei die Öffnung 9 derart ausgebildet ist, dass ein Untersuchungsobjekt entlang einer Systemachse SA der Gantry 20 in die Öffnung 9 eingeführt werden kann, wobei die Translationsbewegung des ersten Gantryteils 21 relativ zu dem zweiten Gantryteil 22 entlang der Systemachse SA erfolgt. Insbesondere ist vorgesehen, dass der erste Gantryteil 21 im Wesentlichen ringförmig um die Systemachse SA herum angeordnet ist und/oder dass der dritte Gantryteil 23 im Wesentlichen ringförmig um die Systemachse SA herum angeordnet ist.

Der erste Gantryteil 21 weist eine Rückseite einer Verkleidung V der Gantry 20 auf, wobei die Rückseite der Verkleidung V der Gantry 20 eine Rückseite der Öffnung 9 ringförmig umgibt. Der dritte Gantryteil 23 weist eine Vorderseite einer Verkleidung V der Gantry 20 auf, wobei die Vorderseite der Verkleidung V der Gantry 20 eine Vorderseite der Öffnung 9 ringförmig umgibt. Die vierte Verbindungseinheit C4 ist an der Vorderseite der Verkleidung V der Gantry 20 befestigt.

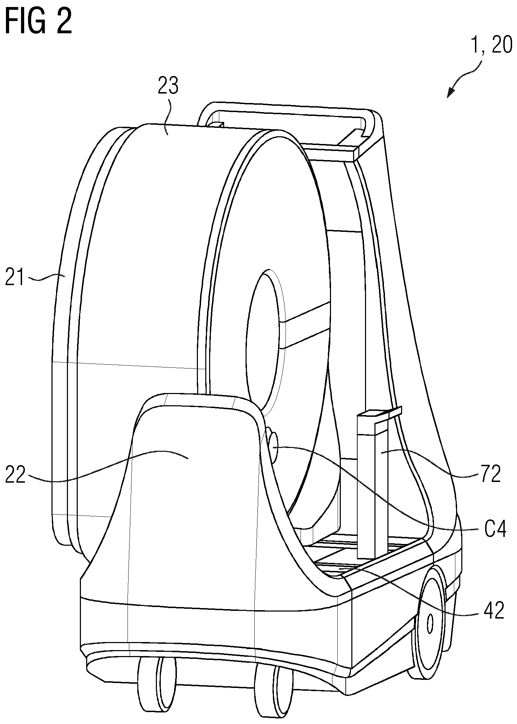

Die Fig. 2 zeigt ein Computertomographiegerät 1 in einem ersten Betriebszustand. In dem ersten Betriebszustand befinden sich der erste Gantryteil 21 und der dritte Gantryteil 23 am hinteren Ende der Gantry 20. Dadurch steht mehr Platz an der Vorderseite der Gantry 20 für eine Positionierung eines Untersuchungsobjekts zu Verfügung.

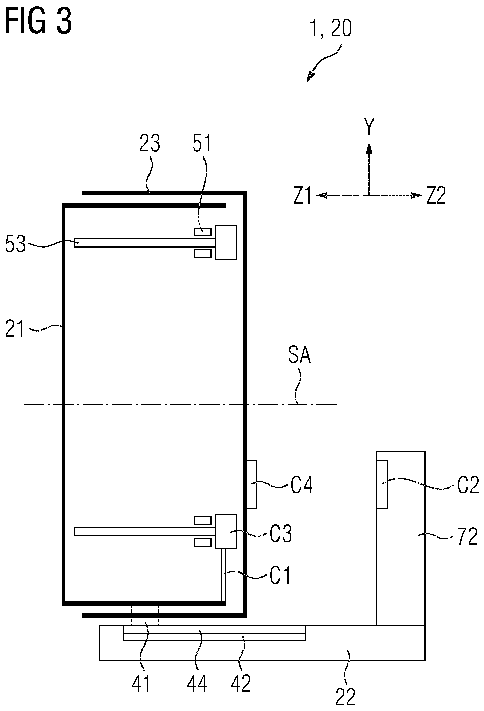

Die Fig. 3 zeigt eine Schnittansicht des Computertomographiegeräts 1 in dem ersten Betriebszustand. In dem ersten Betriebszustand ist die erste Verbindung hergestellt und die zweite Verbindung gelöst, sodass der dritte Gantryteil 23 der Translationsbewegung des ersten Gantryteils 21 relativ zu dem zweiten Gantryteil 22 folgt und insbesondere während der Translationsbewegung des ersten Gantryteils 21 relativ zu dem zweiten Gantryteil 22 relativ zu dem ersten Gantryteil 21 ruht. Der erste Gantryteil 21 weist eine erste Verbindungseinheit C1 auf. Der zweite Gantryteil 22 weist eine zweite Verbindungseinheit C2 auf.

Der dritte Gantryteil 23 weist eine dritte Verbindungseinheit C3 auf, die derart zu der ersten Verbindungseinheit C1 korrespondierend ausgebildet ist, dass mittels der ersten Verbindungseinheit C1 und der dritten Verbindungseinheit C3 die erste Verbindung hergestellt werden kann, welche den dritten Gantryteil 23 relativ zu dem ersten Gantryteil 21 lösbar fixiert, wobei der dritte Gantryteil 23 der Translationsbewegung des ersten Gantryteils 21 relativ zu dem zweiten Gantryteil 22 folgt, wenn die erste Verbindung hergestellt ist.

Der dritte Gantryteil 23 weist eine vierte Verbindungseinheit C4 auf, die derart zu der zweiten Verbindungseinheit C2 korrespondierend ausgebildet ist, dass mittels der zweiten Verbindungseinheit C2 und der vierten Verbindungseinheit C4 die zweite Verbindung hergestellt werden kann, welche den dritten Gantryteil 23 relativ zu dem zweiten Gantryteil 22 lösbar fixiert, wobei während der Translationsbewegung des ersten Gantryteils 21 relativ zu dem zweiten Gantryteil 22 der dritte Gantryteil 23 relativ zu dem zweiten Gantryteil 22 ruht, wenn die erste Verbindung gelöst ist und die zweite Verbindung hergestellt ist.

Die erste Linearführung kann beispielsweise ein Schienensystem 42 und ein Wagensystem 41, welches mit dem Schienensystem 42 zusammenwirkt, aufweisen. Insbesondere kann vorgesehen sein, dass der erste Gantryteil 21 das Wagensystem 41 aufweist und/oder dass der zweite Gantryteil 22 das Schienensystem 42 aufweist. Die Gantry 20 kann beispielsweise ferner einen Linearantrieb 44 zum Antreiben der Translationsbewegung des ersten Gantryteils 21 relativ zu dem zweiten Gantryteil 22 aufweisen.

Der zweite Gantryteil 22 weist die Haltevorrichtung 72 auf. An der Haltevorrichtung 72 können das Schulterbrett 72 und die zweite Verbindungseinheit C2 befestigt werden. Die Haltevorrichtung 72 erstreckt sich entlang der vertikalen Richtung Y. Ein möglichst kleiner Abstand der zweiten Verbindungseinheit C2 und der vierten Verbindungseinheit C4 zur Systemachse SA ist vorteilhaft, um ein Verkanten des dritten Gantryteils 23 zu verringern, wenn über die zweite Verbindung eine Zugkraft auf das dritte Gantryteil 23 ausgeübt wird. Die vierte Verbindungseinheit C4 befindet sich in Bezug auf die Systemachse SA zwischen dem ersten Gantryteil 21 und der zweiten Verbindungseinheit C2. Die Vorderseite der Verkleidung V der Gantry 20 erstreckt sich in einer Frontalebene, die zu der Systemachse SA im Wesentlichen senkrecht ist.

Der dritte Gantryteil 23 ist mittels einer zweiten Linearführung relativ zu dem ersten Gantryteil 21 bewegbar gelagert, wobei die zweite Linearführung parallel zu der ersten Linearführung ausgerichtet ist. Die erste Verbindung basiert auf einer Verriegelung, welche den dritten Gantryteil 23 formschlüssig gegen eine Translationsbewegung des dritten Gantryteils 23 entlang der zweiten Linearführung relativ zu dem ersten Gantryteil 21 sichert.

Die zweite Linearführung weist einen Satz von zueinander parallelen Führungswellen 53 und einen Satz von Kugelbuchsen 51 auf, wobei jede Führungswelle des Satzes von zueinander parallelen Führungswellen 53 in einer entsprechenden Kugelbuchse des Satzes von Kugelbuchsen 51 für eine Längsbewegung dieser Führungswelle gelagert ist. Insbesondere ist vorgesehen, dass der erste Gantryteil 21 den Satz von Kugelbuchsen 51 aufweist und dass der dritte Gantryteil 23 den Satz von zueinander parallelen Führungswellen 53 aufweist.

Insbesondere ist vorgesehen, dass die erste Linearführung parallel zu der Systemachse SA ausgerichtet ist und/oder dass die zweite Linearführung parallel zu der Systemachse SA ausgerichtet ist. Insbesondere kann vorgesehen sein, dass jede Führungswelle des Satzes von zueinander parallelen Führungswellen 53 zu der Systemachse SA parallel ist und/oder dass für jede Führungswelle des Satzes von zueinander parallelen Führungswellen 53 die Längsbewegung dieser Führungswelle entlang der Systemachse SA erfolgt.

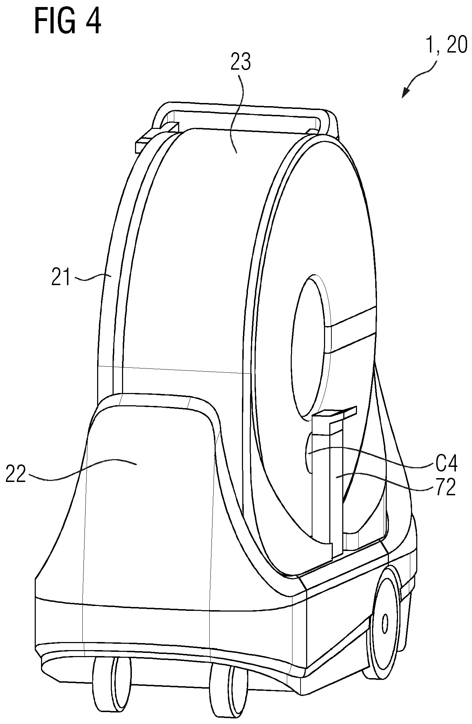

Die Fig. 4 zeigt das Computertomographiegerät 1 in einem zweiten Betriebszustand. In dem zweiten Betriebszustand befinden sich der erste Gantryteil 21 und der dritte Gantryteil 23 am vorderen Ende der Gantry 20. Dadurch befindet sich das Projektionsdatenakquisitionssystem 27 in einer Start-Position einer Scanbewegung.

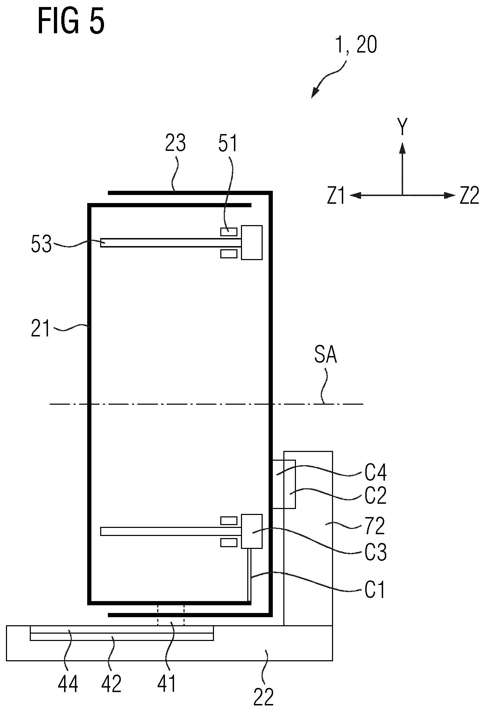

Die Fig. 5 zeigt eine Schnittansicht des Computertomographiegeräts 1 in einem Zwischenzustand. In dem Zwischenzustand ist die erste Verbindung hergestellt und die zweite Verbindung hergestellt. Das Computertomographiegerät 1 befindet sich somit zwischen dem ersten Betriebszustand und dem zweiten Betriebszustand.

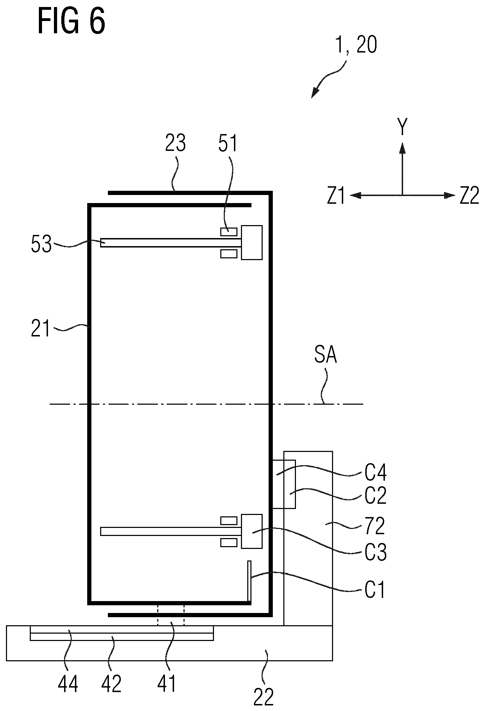

Die Fig. 6 zeigt eine Schnittansicht des Computertomographiegeräts 1 in dem zweiten Betriebszustand. In dem zweiten Betriebszustand ist die erste Verbindung gelöst und die zweite Verbindung hergestellt, sodass während der Translationsbewegung des ersten Gantryteils 21 relativ zu dem zweiten Gantryteil 22 der dritte Gantryteil 23 relativ zu dem zweiten Gantryteil 22 ruht.

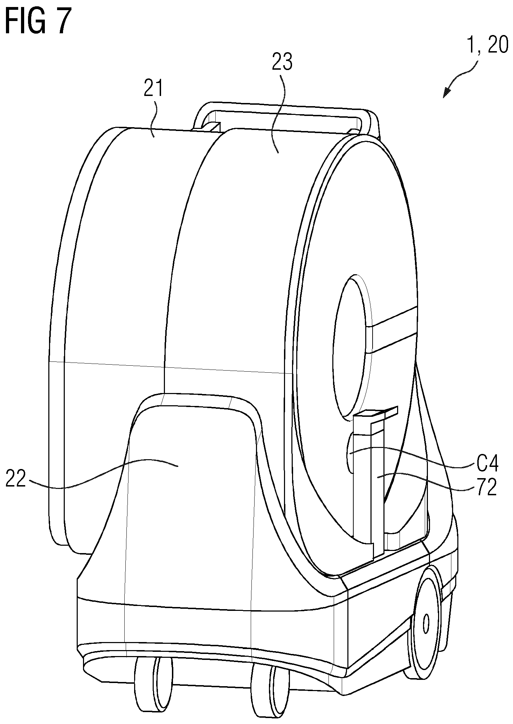

Die Fig. 7 zeigt das Computertomographiegerät 1 in einem dritten Betriebszustand. In dem dritten Betriebszustand befindet sich der erste Gantryteil 21 am hinteren Ende der Gantry 20 und der dritte Gantryteil 23 am vorderen Ende der Gantry 20.

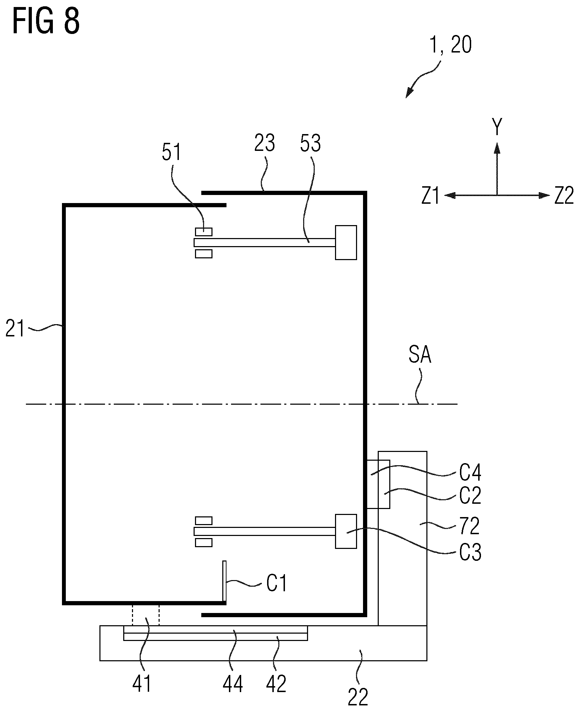

Die Fig. 8 zeigt eine Schnittansicht des Computertomographiegeräts 1 in dem dritten Betriebszustand. In dem dritten Betriebszustand ist die erste Verbindung gelöst und die zweite Verbindung hergestellt, sodass während der Translationsbewegung des ersten Gantryteils 21 relativ zu dem zweiten Gantryteil 22 der dritte Gantryteil 23 relativ zu dem zweiten Gantryteil 22 ruht.

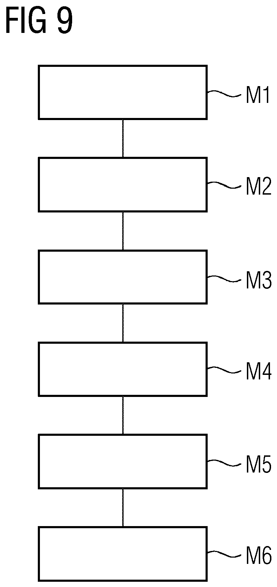

Die Fig. 9 zeigt ein Ablaufdiagramm eines Verfahrens zum Ausführen von Translationsbewegungen von Gantryteilen eines Computertomographiegeräts 1, das Verfahren umfassend:
- ein Herstellen M1 der ersten Verbindung,
- ein erstes Ausführen M2 der Translationsbewegung des ersten Gantryteils 21 relativ zu dem zweiten Gantryteil 22 in eine erste Richtung Z1, wobei der dritte Gantryteil 23 der Translationsbewegung des ersten Gantryteils 21 relativ zu dem zweiten Gantryteil 22 folgt,
- ein erstes Ausführen M3 der Translationsbewegung des ersten Gantryteils 21 relativ zu dem zweiten Gantryteil 22 in eine zweite Richtung Z2, wobei der dritte Gantryteil 23 der Translationsbewegung des ersten Gantryteils 21 relativ zu dem zweiten Gantryteil 22 folgt,
- ein Herstellen M4 der zweiten Verbindung,
- ein Lösen M5 der ersten Verbindung,
- ein zweites Ausführen M6 der Translationsbewegung des ersten Gantryteils 21 relativ zu dem zweiten Gantryteil 22 in die erste Richtung Z1, wobei der dritte Gantryteil 23 relativ zu dem zweiten Gantryteil 22 ruht.

Insbesondere kann die zweite Richtung Z2 zu der ersten Richtung Z1 entgegengesetzt sein. Insbesondere kann vorgesehen sein, dass die erste Richtung Z1 zu der Systemachse SA parallel ist und/oder dass die zweite Richtung Z2 zu der Systemachse SA parallel ist.

## Patentansprüche

1. Computertomographiegerät (1), aufweisend eine Gantry (20) mit einem ersten Gantryteil (21), einem zweiten Gantryteil (22) und einem dritten Gantryteil (23),
- wobei die Gantry (20) eine Öffnung (9) aufweist, wobei die Öffnung (9) derart ausgebildet ist, dass ein Untersuchungsobjekt entlang einer Systemachse (SA) der Gantry (20) in die Öffnung (9) eingeführt werden kann,
- wobei der erste Gantryteil (21) eine Rückseite einer Verkleidung (V) der Gantry (20) aufweist, wobei die Rückseite der Verkleidung (V) der Gantry (20) eine Rückseite der Öffnung (9) ringförmig umgibt, wobei der dritte Gantryteil (23) eine Vorderseite einer Verkleidung (V) der Gantry (20) aufweist, wobei die Vorderseite der Verkleidung (V) der Gantry (20) eine Vorderseite der Öffnung (9) ringförmig umgibt,
- wobei der erste Gantryteil (21) einen Rotor (24) mit einem Projektionsdatenakquisitionssystem (27) aufweist und mittels einer ersten Linearführung derart relativ zu dem zweiten Gantryteil (22) bewegbar gelagert ist, dass eine Translationsbewegung des ersten Gantryteils (21) relativ zu dem zweiten Gantryteil (22) ausgeführt werden kann, wobei die Translationsbewegung des ersten Gantryteils (21) relativ zu dem zweiten Gantryteil (22) entlang der Systemachse (SA) erfolgt, wobei die erste Linearführung ein Schienensystem (42) und ein Wagensystem (41), welches mit dem Schienensystem (42) zusammenwirkt, aufweist, wobei der erste Gantryteil (21) das Wagensystem (41) aufweist und der zweite Gantryteil (22) das Schienensystem (42) aufweist,
- wobei der erste Gantryteil (21) eine erste Verbindungseinheit (C1) aufweist und der zweite Gantryteil (22) eine zweite Verbindungseinheit (C2) aufweist,
- wobei der dritte Gantryteil (23) eine dritte Verbindungseinheit (C3) aufweist, die derart zu der ersten Verbindungseinheit (C1) korrespondierend ausgebildet ist, dass mittels der ersten Verbindungseinheit (C1) und der dritten Verbindungseinheit (C3) eine erste Verbindung hergestellt werden kann, welche den dritten Gantryteil (23) relativ zu dem ersten Gantryteil (21) lösbar fixiert, wobei der dritte Gantryteil (23) der Translationsbewegung des ersten Gantryteils (21) relativ zu dem zweiten Gantryteil (22) folgt, wenn die erste Verbindung hergestellt ist,
- wobei der dritte Gantryteil (23) eine vierte Verbindungseinheit (C4) aufweist, die derart zu der zweiten Verbindungseinheit (C2) korrespondierend ausgebildet ist, dass mittels der zweiten Verbindungseinheit (C2) und der vierten Verbindungseinheit (C4) eine zweite Verbindung hergestellt werden kann, welche den dritten Gantryteil (23) relativ zu dem zweiten Gantryteil (22) lösbar fixiert, wobei während der Translationsbewegung des ersten Gantryteils (21) relativ zu dem zweiten Gantryteil (22) der dritte Gantryteil (23) relativ zu dem zweiten Gantryteil (22) ruht, wenn die erste Verbindung gelöst ist und die zweite Verbindung hergestellt ist,
- **dadurch gekennzeichnet, dass** der dritte Gantryteil (23) mittels einer zweiten Linearführung relativ zu dem ersten Gantryteil (21) bewegbar gelagert ist, wobei die zweite Linearführung parallel zu der ersten Linearführung ausgerichtet ist, wobei die zweite Linearführung einen Satz von zueinander parallelen Führungswellen (53) und einen Satz von Kugelbuchsen (51) aufweist, wobei jede Führungswelle des Satzes von zueinander parallelen Führungswellen (53) in einer entsprechenden Kugelbuchse des Satzes von Kugelbuchsen (51) für eine Längsbewegung dieser Führungswelle gelagert ist.

2. Computertomographiegerät (1) nach Anspruch 1,
- wobei die erste Verbindung formschlüssig ist und/oder
- wobei die zweite Verbindung kraftschlüssig ist.

3. Computertomographiegerät (1) nach Anspruch 1 oder 2,
- wobei die zweite Verbindung auf einer magnetischen Anziehung zwischen der zweiten Verbindungseinheit (C2) und der vierten Verbindungseinheit (C4) basiert.

4. Computertomographiegerät (1) nach einem der Ansprüche 1 bis 3,
- wobei die zweite Verbindungseinheit (C2) einen Elektromagneten aufweist,
- wobei die vierte Verbindungseinheit (C4) ein ferromagnetisches Material aufweist.

5. Computertomographiegerät (1) nach einem der Ansprüche 1 bis 4,
- wobei die erste Verbindungseinheit (C1) einen Riegel und einen Aktor zum Verschieben des Riegels aufweist,
- wobei die dritte Verbindungseinheit (C3) zur formschlüssigen Aufnahme des Riegels eingerichtet ist.

6. Computertomographiegerät (1) nach einem der Ansprüche 1 bis 5,
- wobei der erste Gantryteil (21) den Satz von Kugelbuchsen (51) aufweist und der dritte Gantryteil (23) den Satz von zueinander parallelen Führungswellen (53) aufweist.

7. Computertomographiegerät (1) nach einem der Ansprüche 1 bis 6,
- wobei die erste Verbindung auf einer Verriegelung basiert, welche den dritten Gantryteil (23) formschlüssig gegen eine Translationsbewegung des dritten Gantryteils (23) entlang der zweiten Linearführung relativ zu dem ersten Gantryteil (21) sichert.

8. Computertomographiegerät (1) nach einem der Ansprüche 1 bis 7,
- wobei die zueinander parallelen Führungswellen (53) des Satzes von zueinander parallelen Führungswellen (53) um die Systemachse (SA) herum in einem im Wesentlichen gleichmäßigen auf die Systemachse (SA) bezogenen Winkelabstand voneinander angeordnet sind.

9. Computertomographiegerät (1) nach einem der Ansprüche 1 bis 8,
- wobei der dritte Gantryteil (23) wenigstens eine Führungswelle des Satzes von zueinander parallelen Führungswellen (53) aufweist, welche eine Einkerbung zur formschlüssigen Aufnahme eines entsprechend angeordneten Riegels der ersten Verbindungseinheit (C1) aufweist.

10. Computertomographiegerät (1) nach einem der Ansprüche 1 bis 9,
- wobei die vierte Verbindungseinheit (C4) an der Vorderseite der Verkleidung (V) der Gantry (20) befestigt ist.

11. Verfahren zum Ausführen von Translationsbewegungen von Gantryteilen eines Computertomographiegeräts (1) nach einem der Ansprüche 1 bis 10, das Verfahren umfassend:
- ein Herstellen (M1) der ersten Verbindung,
- ein erstes Ausführen (M2) der Translationsbewegung des ersten Gantryteils (21) relativ zu dem zweiten Gantryteil (22) in eine erste Richtung (Z1), wobei der dritte Gantryteil (23) der Translationsbewegung des ersten Gantryteils (21) relativ zu dem zweiten Gantryteil (22) folgt,
- ein erstes Ausführen (M3) der Translationsbewegung des ersten Gantryteils (21) relativ zu dem zweiten Gantryteil (22) in eine zweite Richtung (Z2), wobei der dritte Gantryteil (23) der Translationsbewegung des ersten Gantryteils (21) relativ zu dem zweiten Gantryteil (22) folgt,
- ein Herstellen (M4) der zweiten Verbindung,
- ein Lösen (M5) der ersten Verbindung,
- ein zweites Ausführen (M6) der Translationsbewegung des ersten Gantryteils (21) relativ zu dem zweiten Gantryteil (22) in die erste Richtung (Z1), wobei der dritte Gantryteil (23) relativ zu dem zweiten Gantryteil (22) ruht.

12. Verfahren nach Anspruch 11,
- wobei die erste Verbindungseinheit (C1) einen Riegel und einen Aktor zum Verschieben des Riegels aufweist,
- wobei die dritte Verbindungseinheit (C3) zur formschlüssigen Aufnahme des Riegels eingerichtet ist,
- wobei die erste Verbindung hergestellt wird, indem der Riegel mittels des Aktors hin zu der dritten Verbindungseinheit (C3) verschoben und mittels der dritten Verbindungseinheit (C3) formschlüssig aufgenommen wird,
- wobei die erste Verbindung gelöst wird, indem der Riegel mittels des Aktors weg von der dritten Verbindungseinheit (C3) verschoben wird.

13. Verfahren nach Anspruch 11 oder 12,
- wobei die zweite Verbindungseinheit (C2) einen Elektromagneten aufweist,
- wobei die vierte Verbindungseinheit (C4) ein ferromagnetisches Material aufweist,
- wobei durch das erste Ausführen (M3) der Translationsbewegung des ersten Gantryteils (21) relativ zu dem zweiten Gantryteil (22) in die zweite Richtung (Z2) das ferromagnetische Material der vierten Verbindungseinheit (C4) in einen Wirkungsbereich des Elektromagneten gebracht wird, während der Elektromagnet ausgeschaltet ist,
- wobei die zweite Verbindung hergestellt wird, indem der Elektromagnet eingeschaltet wird.

## Claims

1. Computed tomography device (1), having a gantry (20) with a first gantry part (21), a second gantry part (22) and a third gantry part (23),
- wherein the gantry (20) has an opening (9), wherein the opening (9) is designed such that an object under examination can be introduced into the opening (9) along a system axis (SA) of the gantry (20),
- wherein the first gantry part (21) has a rear side of a casing (V) of the gantry (20), wherein the rear side of the casing (V) of the gantry (20) annularly surrounds a rear side of the opening (9), wherein the third gantry part (23) has a front side of a casing (V) of the gantry (20), wherein the front side of the casing (V) of the gantry (20) annularly surrounds a front side of the opening (9),
- wherein the first gantry part (21) has a rotor (24) with a projection data acquisition system (27) and by means of a first linear guide is movably mounted relative to the second gantry part (22) such that a translation movement of the first gantry part (21) relative to the second gantry part (22) can be executed, wherein the translation movement of the first gantry part (21) relative to the second gantry part (22) takes place along the system axis (SA), wherein the first linear guide has a rail system (42) and a trolley system (41) which interacts with the rail system (42), wherein the first gantry part (21) has the trolley system (41) and the second gantry part (22) has the rail system (42),
- wherein the first gantry part (21) has a first connection unit (C1) and the second gantry part (22) has a second connection unit (C2),
- wherein the third gantry part (23) has a third connection unit (C3), which is designed to correspond to the first connection unit (C1), such that by means of the first connection unit (C1) and the third connection unit (C3) a first connection can be created, which detachably fixes the third gantry part (23) relative to the first gantry part (21), wherein the third gantry part (23) follows the translation movement of the first gantry part (21) relative to the second gantry part (22) if the first connection is created,
- wherein the third gantry part (23) has a fourth connection unit (C4), which is designed to correspond to the second connection unit (C2), such that by means of the second connection unit (C2) and the fourth connection unit (C4) a second connection can be created, which detachably fixes the third gantry part (23) relative to the second gantry part (22), wherein during the translation movement of the first gantry part (21) relative to the second gantry part (22) the third gantry part (23) is at rest relative to the second gantry part (22) if the first connection is released and the second connection is created,
- **characterised in that** the third gantry part (23) is movably mounted relative to the first gantry part (21) by means of a second linear guide, wherein the second linear guide is aligned in parallel to the first linear guide, wherein the second linear guide has a set of parallel guide shafts (53) and a set of ball bushings (51), wherein each guide shaft of the set of parallel guide shafts (53) is mounted in a corresponding ball bushing of the set of ball bushings (51) for a longitudinal movement of this guide shaft.

2. Computed tomography device (1) according to claim 1,
- wherein the first connection is form-fit and/or
- wherein the second connection is force-fit.

3. Computed tomography device (1) according to claim 1 or 2,
- wherein the second connection is based on a magnetic attraction between the second connection unit (C2) and the fourth connection unit (C4).

4. Computed tomography device (1) according to one of claims 1 to 3,
- wherein the second connection unit (C2) has an electromagnet,
- wherein the fourth connection unit (C4) has a ferromagnetic material.

5. Computed tomography device (1) according to one of claims 1 to 4,
- wherein the first connection unit (C1) has a bolt and an actuator for displacing the bolt,
- wherein the third connection unit (C3) is designed for the form-fit reception of the bolt.

6. Computed tomography device (1) according to one of claims 1 to 5,
- wherein the first gantry part (21) has the set of ball bushings (51) and the third gantry part (23) has the set of parallel guide shafts (53).

7. Computed tomography device (1) according to one of claims 1 to 6,
- wherein the first connection is based on a lock, which secures the third gantry part (23) in a form-fit manner against a translation movement of the third gantry part (23) along the second linear guide relative to the first gantry part (21).

8. Computed tomography device (1) according to one of claims 1 to 7,
- wherein the parallel guide shafts (53) of the set of parallel guide shafts (53) are arranged around the system axis (SA) at a substantially uniform angular distance from one another in relation to the system axis (SA).

9. Computed tomography device (1) according to one of claims 1 to 8,
- wherein the third gantry part (23) has at least one guide shaft of the set of parallel guide shafts (53), which has a notch for the form-fit reception of a correspondingly arranged bolt of the first connection unit (C1).

10. Computed tomography device (1) according to one of claims 1 to 9,
- wherein the fourth connection unit (C4) is fastened to the front side of the casing (V) of the gantry (20).

11. Method for executing translation movements of gantry parts of a computed tomography device (1) according to one of claims 1 to 10, the method comprising:
- a creation (M1) of the first connection,
- a first execution (M2) of the translation movement of the first gantry part (21) relative to the second gantry part (22) in a first direction (Z1), wherein the third gantry part (23) follows the translation movement of the first gantry part (21) relative to the second gantry part (22),
- a first execution (M3) of the translation movement of the first gantry part (21) relative to the second gantry part (22) in a second direction (Z2), wherein the third gantry part (23) follows the translation movement of the first gantry part (21) relative to the second gantry part (22),
- a creation (M4) of the second connection,
- a release (M5) of the first connection,
- a second execution (M6) of the translation movement of the first gantry part (21) relative to the second gantry part (22) in the first direction (Z1), wherein the third gantry part (23) is at rest relative to the second gantry part (22).

12. Method according to claim 11,
- wherein the first connection unit (C1) has a bolt and an actuator for displacing the bolt,
- wherein the third connection unit (C3) is designed for the form-fit reception of the bolt,
- wherein the first connection is created by the bolt being displaced by means of the actuator to the third connection unit (C3) and is received in a form-fit manner by means of the third connection unit (C3),
- wherein the first connection is released by the bolt being displaced away from the third connection unit (C3) by means of the actuator.

13. Method according to claim 11 or 12,
- wherein the second connection unit (C2) has an electromagnet,
- wherein the fourth connection unit (C4) has a ferromagnetic material,
- wherein due to the first execution (M3) of the translation movement of the first gantry part (21) relative to the second gantry part (22) in the second direction (Z2) the ferromagnetic material of the fourth connection unit (C4) is brought within an effective range of the electromagnet, while the electromagnet is deactivated,
- wherein the second connection is created by the electromagnet being activated.

## Revendications

1. Appareil (1) de tomodensitométrie assistée par ordinateur, comprenant un portique (20) ayant une première partie (21) de portique, une deuxième partie (22) de portique et une troisième partie (23) de portique,
- dans lequel le portique (20) a une ouverture (9), dans lequel l'ouverture (9) est constituée de manière à pouvoir introduire un objet à examiner dans l'ouverture (9) suivant un axe (SA) de système du portique (20),
- dans lequel la première partie (21) du portique a un côté arrière d'un habillage (V) du portique (20), dans lequel le côté arrière de l'habillage (V) du portique (20) entoure annulairement un côté arrière de l'ouverture (9), dans lequel la troisième partie (23) du portique a un côté avant d'un habillage (V) du portique (20), dans lequel le côté avant de l'habillage (V) du portique (20) entoure annulairement un côté avant de l'ouverture (9),
- dans lequel la première partie (21) du portique a un rotor (24) ayant un système (27) d'acquisition de données de projection et est montée mobile par rapport à la deuxième partie (22) du portique au moyen d'un premier guidage linéaire, de manière à pouvoir exécuter un déplacement en translation de la première partie (21) du portique par rapport à la deuxième partie (22) du portique, dans lequel le mouvement en translation de la première partie (21) du portique par rapport à la deuxième partie (22) du portique a lieu suivant l'axe (SA) du système, dans lequel le premier guidage linéaire a un système (42) de rail et un système (41) de chariot, qui coopère avec le système (42) de rail, dans lequel la première partie (21) du portique a le système (41) de chariot et la deuxième partie (22) du portique a le système (42) de rail,
- dans lequel la première partie (21) du portique a une première unité (C1) de liaison et la deuxième partie (22) du portique a une deuxième unité (C2) de liaison,
- dans lequel la troisième partie (23) du portique a une troisième unité (C3) de liaison, qui est constituée d'une manière correspondante à la première unité (C1) de liaison, de manière à pouvoir, au moyen de la première unité (C1) de liaison et de la troisième unité (C3) de liaison, produire une première liaison, qui immobilise de manière amovible, la troisième partie (23) du portique par rapport à la première partie (21) du portique, dans lequel la troisième partie (23) du portique suit le mouvement en translation de la première partie (21) du portique par rapport à la deuxième partie (22) du portique, lorsque la première liaison est produite,
- dans lequel la troisième partie (23) du portique a une quatrième unité (C4) de liaison, qui est constituée d'une manière correspondante à la deuxième unité (C2) de liaison, de manière à pouvoir, au moyen de la deuxième unité (C2) de liaison et de la quatrième unité (C4) de liaison, produire une deuxième liaison, qui immobilise, de manière amovible, la troisième partie (23) de portique, par rapport à la deuxième partie (22) de portique, dans lequel, pendant le mouvement en translation de la première partie (21) du portique par rapport à la deuxième partie (22) du portique, la troisième partie (23) du portique est au repos par rapport à la deuxième partie (22) du portique, lorsque la première liaison est défaite et la deuxième liaison est produite,
- **caractérisé en ce que** la troisième partie (23) du portique est montée, au moyen d'un deuxième guidage linéaire, mobile par rapport à la première partie (21) du portique, dans lequel le deuxième guidage linéaire est dirigé parallèlement au premier guidage linéaire, dans lequel le deuxième guidage linéaire a un ensemble d'arbres (53) de guidage parallèles entre eux et un ensemble de nipples (51), dans lequel chaque arbre de guidage de l'ensemble d'arbres (53) de guidage parallèles entre eux est monté dans un nipple correspondant de l'ensemble de nipples (51) pour un déplacement longitudinal de cet arbre de guidage.

2. Appareil (1) de tomodensitométrie assistée par ordinateur suivant la revendication 1,
- dans lequel la première liaison est à complémentarité de forme et/ou
- dans lequel la deuxième liaison est à coopération de force.

3. Appareil (1) de tomodensitométrie assistée par ordinateur suivant la revendication 1 ou 2,
- dans lequel la deuxième liaison repose sur une attraction magnétique entre la deuxième unité (C2) de liaison et la quatrième unité (C4) de liaison.

4. Appareil (1) de tomodensitométrie assistée par ordinateur suivant l'une des revendications 1 à 3,
- dans lequel la deuxième unité (C2) de liaison a un électroaimant,
- dans lequel la quatrième unité (C4) de liaison a du matériau ferromagnétique.

5. Appareil (1) de tomodensitométrie assistée par ordinateur suivant l'une des revendications 1 à 4,
- dans lequel la première unité (C1) de liaison a un verrou et un actionneur pour déplacer le verrou,
- dans lequel la troisième unité (C3) de liaison est agencée pour la réception à complémentarité de forme du verrou.

6. Appareil (1) de tomodensitométrie assistée par ordinateur suivant l'une des revendications 1 à 5,
- dans lequel la première partie (21) du portique a l'ensemble de nipples (51) et la troisième partie (23) du portique a l'ensemble d'arbres (53) de guidage parallèles entre eux.

7. Appareil (1) de tomodensitométrie assistée par ordinateur suivant l'une des revendications 1 à 6,
- dans lequel la première liaison repose sur un verrouillage, assure la troisième partie (23) du portique à complémentarité de forme à l'encontre d'un mouvement en translation de la troisième partie (23) du portique suivant le deuxième guidage linéaire par rapport à la première partie (21) du portique.

8. Appareil (1) de tomodensitométrie assistée par ordinateur suivant l'une des revendications 1 à 7,
- dans lequel les arbres (53) de guidage parallèles entre eux de l'ensemble d'arbres (53) de guidage parallèles entre eux sont disposés autour de l'axe (SA) du système les uns par rapport aux autres à une distance angulaire, rapportée à l'axe (SA) du système, sensiblement égale.

9. Appareil (1) de tomodensitométrie assistée par ordinateur suivant la revendication 1 à 8,
- dans lequel la troisième partie (23) du portique a au moins un arbre de guidage de l'ensemble d'arbres (53) de guidage parallèles entre eux, qui a une encoche pour la réception à complémentarité de forme d'un verrou, disposé de manière correspondante, de la première unité (C1) de liaison.

10. Appareil (1) de tomodensitométrie assistée par ordinateur suivant l'une des revendications 1 à 9,
- dans lequel l'unité (C4) de liaison est fixée au côté avant de l'habillage (V) du portique (20).

11. Procédé d'exécution de mouvements en translation de parties de portique d'un appareil (1) de tomodensitométrie assistée par ordinateur suivant l'une des revendications 1 à 10, le procédé comprenant :
- une production (M1) de la première liaison,
- une première exécution (M2) du mouvement en translation de la première partie (21) du portique par rapport à la deuxième partie (22) du portique dans une première direction (Z1), dans lequel la troisième partie (23) du portique suit le mouvement en translation de la première partie (21) du portique par rapport à la deuxième partie (22) du portique,
- une première exécution (M3) du mouvement en translation de la première partie (21) du portique par rapport à la deuxième partie (22) du portique dans une deuxième direction (Z2), dans lequel la troisième partie (23) du portique suit le mouvement en translation de la première partie (21) du portique par rapport à la deuxième partie (22) du portique,
- une production (M4) de la deuxième liaison,
- une déliaison (M5) de la première liaison,
- une deuxième exécution (M6) du mouvement en translation de la première partie (21) du portique par rapport à la deuxième partie (22) du portique dans la première direction (Z1), dans laquelle la troisième partie (23) du portique est au repos par rapport à la deuxième partie (22) du portique.

12. Procédé suivant la revendication 11,
- dans lequel la première unité (C1) de liaison a un verrou et un actionneur pour déplacer le verrou,
- dans lequel la troisième unité (C3) de liaison est agencée pour la réception à complémentarité de forme du verrou,
- dans lequel on produit la première liaison en déplaçant le verrou au moyen de l'actionneur vers la troisième unité (C3) de liaison et en le recevant à complémentarité de forme au moyen de la troisième unité (C3) de liaison,
- dans lequel on défait la première liaison en déplaçant le verrou au moyen de l'actionneur en l'éloignant de la troisième unité (C3) de liaison.

13. Procédé suivant la revendication 11 ou 12,
- dans lequel la deuxième unité (C2) de liaison a un électroaimant,
- dans lequel la quatrième unité (C4) de liaison a du matériau ferromagnétique,
- dans lequel, par la première exécution (M3) du mouvement en translation de la première partie (21) du portique par rapport à la deuxième partie (22) du portique dans la deuxième direction (Z2), on met le matériau ferromagnétique de la quatrième unité (C4) de liaison dans une zone d'action de l'électroaimant, alors que l'électroaimant est mis hors circuit,
- dans lequel on produit la deuxième liaison en mettant l'électroaimant en circuit.
